# EUROPEAN PATENT SPECIFICATION

(11) **EP 2 269 543 B1**
(45) Date of publication and mention of the grant of the patent: **19.12.2012**
(21) Application number: 09164418.7
(22) Date of filing: 02.07.2009
(51) Int. Cl.: A61F 5/56

(54) **Device for mandibular advancement**
Vorrichtung zur Mandibularentwicklung
Dispositif pour avancement mandibulaire

(43) Date of publication of application: 05.01.2011
(73) Proprietor: Carash Industries Ltd, Worthing Sussex BN14 7QI (GB)
(72) Inventor: Ash, Simon, Worthing, Sussex BN14 7QI (GB)
(74) Representative: Haley, Stephen

(56) References cited:
- WO-A-03/034957
- WO-A-2008/061725
- DE-A1- 10 331 531
- FR-A- 2 887 140
- US-A1- 2007 224 567

## Description

The present invention relates to a device for advancing a lower jaw relative to an upper jaw a predetermined distance and maintaining said distance.

Snoring and sleep apnea are chronic conditions which, in many cases, it would be extremely desirable to manage, without recourse to surgery. Mandibular advancement is recognised as one possible way of treating a patient suffering such a condition.

To avoid soft tissue surgery to the mouth, throat, soft palate or surgical advancement of the lower jaw, nocturnal transitory mandibular advancement devices have been developed and have been shown to be a long term means of managing this condition.

However, this form of treatment commits the patient to wearing an inter-oral appliance indefinitely. Accordingly, it is highly desirable to provide a robust, unobtrusive comfortable device that will be well tolerated by a patient. Such a device should distribute the occlusal and functional loads and forces generated by the jaw protrusion evenly over the teeth, rather than interfere with the soft tissues, that is be tooth borne rather than soft tissue borne, whilst preventing any damage or movement to or of the teeth. Furthermore, it is very desirable that the device does not interfere with, or encroach upon the tongue space. The device should provide forward advancement of the jaw with minimal vertical opening thus reducing lip incompetence and mouth opening often a problem with other mandibular advancement devices described.

Previous devices have been made from acrylic materials, which can often fail due to material weakness as a result of the considerable forces that can be created by the jaws and the bulky nature of these devices. In an attempt to overcome this shortfall, these devices are often bulky and as a result can be uncomfortable to wear and hence fail due to poor patient tolerance. Existing devices generally also require considerable jaw opening, encroach upon tongue space, lead to lip incompetence mouth opening, a dry mouth and other adverse effects for the patient. Essentially, any device or materials that occupy tongue space, and in particular the area between the tongue and the lower teeth, will cause the tongue to rest further back in the mouth, contrary to the required forward placement of the tongue to achieve posterior pharyngeal airway competence. Materials placed in this part of the mouth will also cause the mouth to be opened rather than jaw advancement.

Furthermore, the acrylic material picks up stains, can be difficult to clean, degrade with time often becoming more brittle and liable to distortion with use or if allowed to dry out. In addition, linkage components, such as stainless steel wire, used to link between different members of the devices, are prone to breakage. The metal / acrylic interfaces often show fracture fatigue with use leading to failure. The acrylic or other plastics may break off the devices allowing for uncontrolled and adverse tooth movement/ migration.

The present invention seeks to overcome these and other problems with existing mandibular advancement devices.

FR-A-2887140 discloses an orthodontic device as defined in the preamble of claim 1.

According to the present invention there is provided a device as defined in claim 1.

The present invention is therefore a compact device which is configured to be secured to and rest upon the teeth and is therefore comfortable to wear because it spreads the occlusal loads and forces generated by the jaw protrusion across the teeth in a jaw, which greatly reduces the possibility of damage occurring and limits untoward tooth movements or soft tissue damage.

The present invention uses the thin delicate properties of the cast alloy in the form of a thin palatal plate in the upper jaw plus a connecting bar or other component in the labial sulcus in the lower jaw, leaving the lingual sulcus free of obstruction or tongue encroachment. The device is hard wearing, physically and chemically stable and is thus well tolerated in the mouth. It is very easy to clean and maintain. The cast metal components do not distort or deteriorate with time assuming they are cared for and stored when out of the mouth.

An example of the present invention will now be described with reference to the following figures, in which a device according to the present invention is illustrated as it would be fitted within a patient's mouth, wherein:
Figure 1 shows a device according to the present invention in an open position;
Figure 2 shows the device of figure 1 in a closed position;
Figure 3 shows an occlusal view of the upper member of the device of figure1;
Figure 4 shows an orthogonal view of the lower member of the device of figure 1; and
Figure 5 shows an occlusal view of the lower member of the device of figure 1.

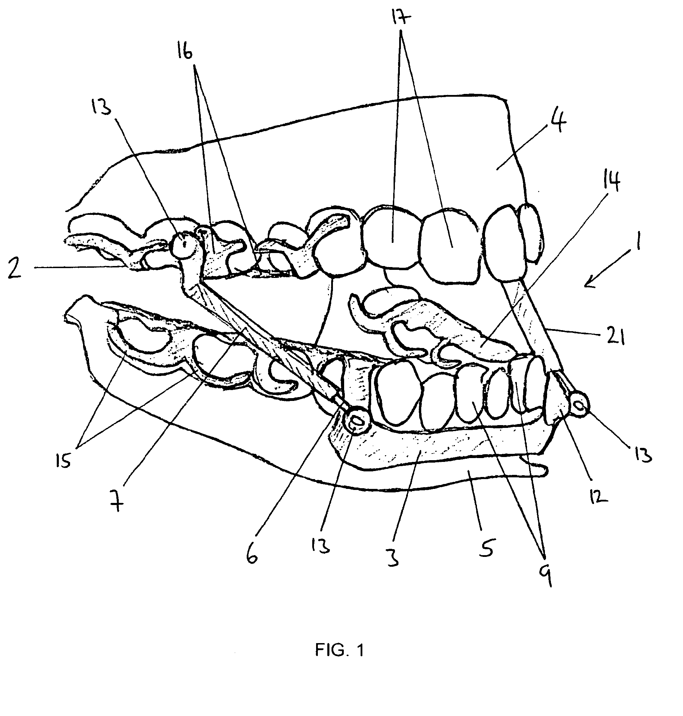

Figure 1 shows an exemplary mandibular device 1 according to the present invention, comprising two main members, an upper member 2 and a lower member 3, which are configured to be fitted into the upper jaw 4 and lower jaw 5, respectively, of a patient's mouth. The upper and lower members 2,3 of the device 1 are connected such that the lower jaw 5 can move relative to the upper jaw 4 when the device 1 is fitted in a patient's mouth, but with the movement of the lower jaw 5 limited such that it is always advanced by a predetermined distance relative to the upper jaw 4.

In the exemplary device shown in the figures contained herein, the upper and lower members 2,3 are connected by a telescopic arm 6 and sleeve 7 arrangement 21, sometimes known as a "Herbst telescope". Of course, it will be appreciated that other suitable connecting means may be used to provide the same function as this arrangement 21, which is simply a preferred connecting means.

The device 1 is formed of a cast metal, for example a gold alloy, but preferably chrome cobalt, which allows the device 1 to be compact but strong.

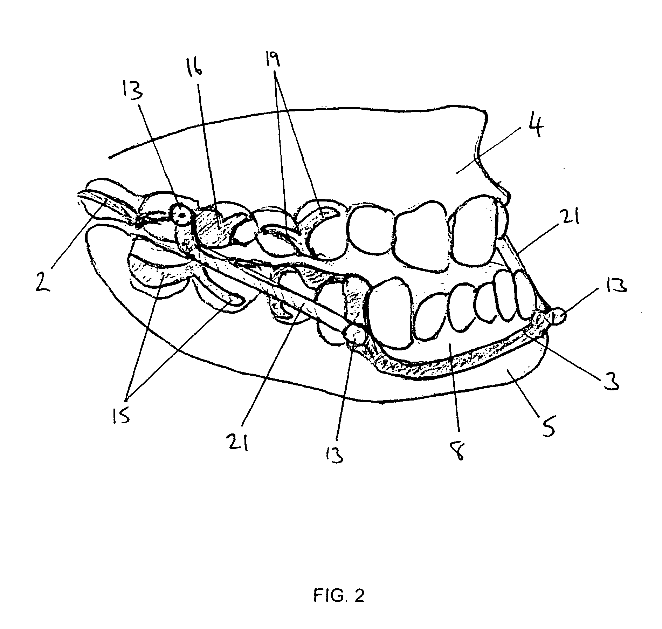

Figure 2 shows the exemplary device 1 in a closed position as it would be fitted in a patient's mouth. It can clearly be seen that the lower member 3 has advanced the lower jaw 5 relative to the upper jaw 4.

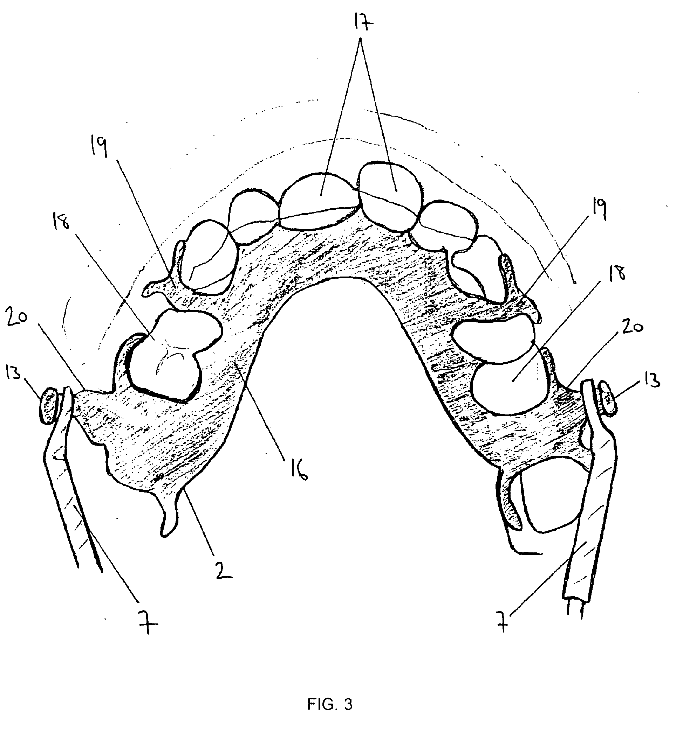

Figure 3 shows an upper member 2 of the exemplary device 1. It can be seen that the upper member 2 is formed as a base plate 16 that fits snugly behind and, ideally, at the base of the upper front teeth 17 in the upper jaw 4. The base plate 16 fits behind the upper front teeth 17 and extends back towards the rearward upper molars 18. Although in the example shown in the figures, the upper member 2 is a single component formed roughly in the shape of a horse shoe, it will, however, be appreciated that an upper member 2 having a different construction, such as having a connecting skeletal arrangement with, for example, a connecting bar, plate or wire between the upper molars 18 on either side of the upper jaw 4, would also perform the same function.

The upper member 2 is secured to the upper jaw 4 by the base plate 16 forming clasps 19 around one or more teeth 18 on either side of the upper jaw 4.

Towards the rearmost upper molars 18 on either side of the upper jaw 4, as the base plate 16 extends backwards, it also extends across the surface of the upper molars 18 so as to cover at least part of the occlusal (biting) surface of the rearward upper molars 18 when fitted to the upper jaw 4. At these points of the upper member 2, mounting points 20 are provided on the buccal (outer) side of the upper molars 18 for attaching the connecting sleeves 7 to. The mounting points 20 are, ideally, an integral part of the upper member 2. As with the connecting arms 6, the connecting sleeves 7 are pivotally and removably attached using, for example, a grub screw 13, although, again, other suitable attaching means could be used.

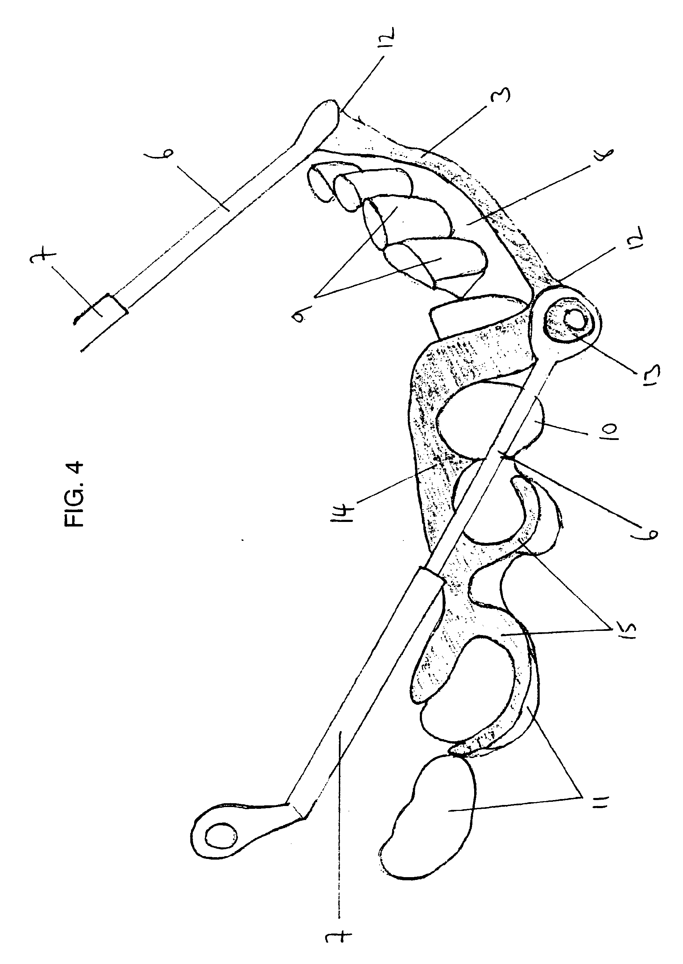

Figure 4 shows the lower member 3 of the exemplary device. It can be seen that the lower member 3 is arranged to be fitted into the lower jaw 5 by placing it in the labial sulcus 8, which is an area of the lower jaw 5 found between the outside of the lower front teeth 9 and the inner surface of the lower lip (not shown). The lower member 3 is configured to extend across substantially the width of the lower front teeth 9 before extending up and backwards so as to cover at least some of the lower cuspid 10 and molar 11 teeth positioned on either side towards the rear of the lower jaw 5. At the points where the lower member 3 begins to extend back toward the rearward molars 11 on either side of the lower jaw 5, which in Figure 4 corresponds roughly to the position of each cuspid, are provided mounting points 12 to which connecting arms 6 are pivotally attached. The mounting points 12 are, ideally, an integral part of the lower member 3, and provide additional strength to the structure. The connecting arm 6 is pivotally and removably attached to the lower member 3 using, for example, a grub screw 13, although other suitable attaching means could be used.

The connecting arm 6 is part of a preferred Herbst telescopic arm 6 and sleeve 7 arrangement 21 and will be described in more detail further on.

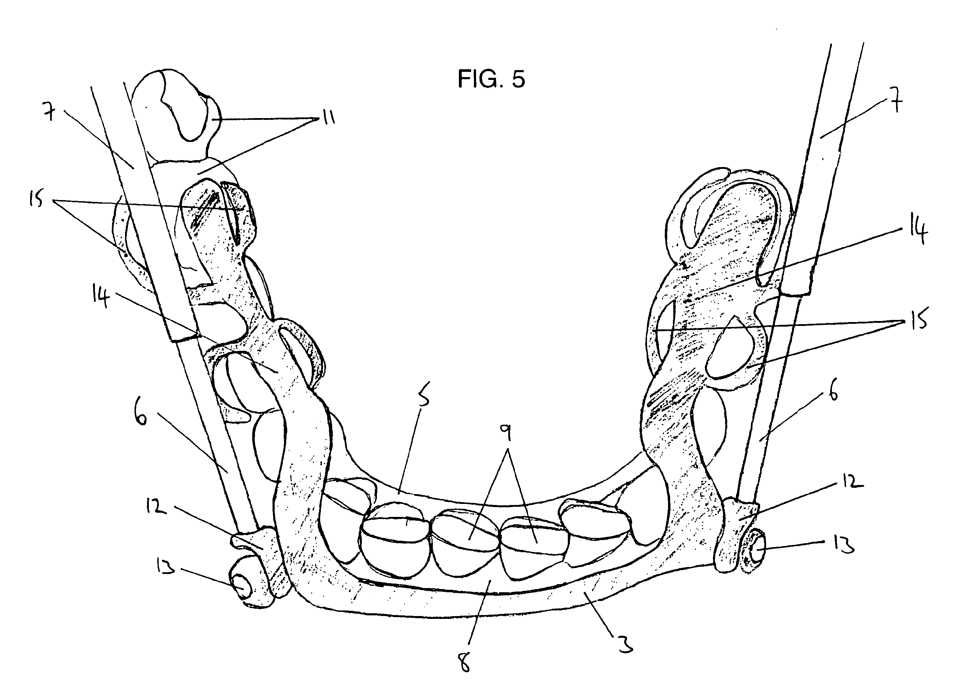

Figure 5 shows that, as the lower member 3 extends backwards so as to cover a part of the rearward lower molars 11 on either side of the lower jaw 5, it forms a protective occlusal base plate 14 on either side of the lower jaw 5 that is arranged to cover at least part of the occlusal surfaces of the molars 11. These base plates 14, and hence the lower member 14, are secured to the lower molars 11, in this example, by way of clasps 15 that secure over and around the buccal (outer) and lingual (inner) surfaces of one or more of the lower molars 11 in a similar manner to how the upper member 2 is attached to the upper molars 18.

A similar arrangement might be that of a lower member 3 comprising two base plates 14 for covering a part of the lower molars on either side of the lower jaw 5, with the base plates 14 being connected by a connecting bar, plate or wire placed in the labial sulcus 8 at the front of the lower jaw 5. Such an arrangement would provide the same function as the exemplary device 1 described herein.

Importantly, once fitted to the lower jaw 5, the lower member 3 should have very little, or no, lateral or antero posterior movement relative to the lower jaw 5. This is because the lower member 3 is secured and anchored to the lower teeth and hence the lower jaw 5.

The telescopic arm 6 and sleeve 7 arrangement 21 of the exemplary device 1 is perhaps best shown in figure 1, with the connecting arm 6 being shown pivotally attached to the lower member 3 and configured to fit inside the connecting sleeve 7 that is pivotally attached to the upper device (not shown), thereby providing a telescopic arm 6 and sleeve 7 arrangement 21.

Of course, it will be appreciated that the arm 6 could alternatively be connected to the upper member 2 and the sleeve 7 connected to the lower member 3, but the configuration shown in the figures is preferred. Furthermore, a skilled person will recognise that although one telescopic arm 6 and sleeve 7 arrangement 21 may perform the invention, two are preferable as they provide more support to the device 1, in use.

The length of the arm 6 is configured such that when the lower member 3 is connected to the upper member 2 by the telescopic arm 6 and sleeve 7 arrangement 21, although the lower member 3 can move relative to the upper member 2, the length of the arm 6 within the sleeve 7 is configured to advance the lower jaw 5 by a predetermined distance, the arm 6 in the sleeve 7 maintaining said minimum distance.

The preferred way to fit the device 1 is first to assemble the upper member 2 and lower member 3 together by inserting the connecting arm 6 into the sleeve 7. Once this has been done the device 1 can then be inserted as a complete unit, into the mouth. The upper member 2 is seated in the upper jaw 4 first, with the clasps 19 securing the upper member 2 to the upper molars 18, and then the lower jaw 5 is advanced to allow the lower member 3 to come to rest over the lower molars 11 and lower jaw 5, where it is secured into place by the clasps 15. The lower jaw 5 will then be maintained at a predetermined advanced position, as explained above.

To remove the device 1, the lower jaw 5 is advanced to allow the lower member 3 to be lifted out and away from their lower teeth before then detaching the upper member 2 from the upper jaw 4. The device 1 can be removed from the mouth as a complete unit.

A skilled person will appreciate that although the upper member 2 and lower member 3 in the example described herein are formed from a single piece of material, they can also be constructed from multiple components.

For example, as previously mentioned, the lower member 3 could be formed from two base plates 14 configured to cover part of the lower molars 11 on either side of the lower jaw 5 and a connecting plate, bar, wire or similar placed in the labial sulcus 8 to connect the base plates 14 together.

Furthermore, the upper member 2 could be formed as a palatal skeleton, that is, as an upper maxillary jaw device having a connecting base plate placed in the maxillary labial sulcus as well as, or in place of, a base plate, or similar, in the palatal area on the inside of the upper teeth.

What the skilled person will, however, appreciate is that an important feature of the present invention is the positioning of the lower member 3 in the labial sulcus 8, or on the outer surface of the lower front teeth 9, which ensures that the tongue space is not encroached

In the above description, the securing means for securing the upper and lower members 2,3 of the device to the upper and lower jaws 4,5, respectively, is described as a clasp 15,19. However, a skilled person will recognise that an important aspect of the securing means of the present invention is that it secures to the teeth in the upper and lower jaws 4,5, and also that it prevents any lateral movement of either the upper or lower members 2,3 relative to the teeth. In the present invention, the contrary displacing forces that are applied to the device, in use, do not follow the designated path of insertion used in the device design and construction.

In addition, although the example described above uses a telescopic arm 6 and sleeve 7 arrangement 21, a skilled person will recognise that the linkage between the upper member 2 and lower member 3 can be either fixed or non fixed. An important feature is that the linkage is positioned in the buccal sulcus portion of the mouth, that is, outside of the teeth, and is configured such that, when fitted, it advances the lower member 3 a predetermined distance, whilst allowing movement of the lower jaw 5 relative to the upper jaw 4. By allowing maximum forward lower jaw 5 placement whilst minimising adverse jaw opening, the present invention enables a wearer to maintain lip competence and reduces the untoward effects of jaw opening and lip incompetence.

The device 1 of the present invention is thinner, smaller and less obtrusive than existing mandibular advancement devices and the use of a cast metal provides a device which is very strong and robust in thin section. Such materials are well tolerated by patients and are inert in the oral environment.

Comfort and patient tolerance is a main concern with such devices and the present invention addresses this. The positioning of the lower member 3 of the device 1 in the labial sulcus 8 in the lower jaw 5, leaves the lingual space free from device encroachment thus maximising jaw and tongue advancement. Furthermore, placing the telescopic linkages in the labial and buccal sulcus further serves to leave the inner oral / lingual space free from encroachment, thus maximising forward advancement of the tongue.

Accordingly, the device 1 does not encroach the tongue space and, furthermore, allows a patient to open their mouth without the need to remove or displace the device 1, thus allowing drinks and nocturnal medication to be taken, or indeed allowing the patient to talk, with the device in situ. Hence the device 1 is extremely comfortable and convenient to wear.

Hither to, no acrylic device which has been devised to advance the mandible has been constructed having a lower member 3 that is positioned in the labial sulcus 8. This unique feature is only possible to achieve if a metal alloy or similar material are used, as described herein.

In addition, the device 1 of the present invention minimises unwanted tooth movements often encountered with non-prescribed or acrylic devices by using base plates 14,16 , which are supported by the teeth and secured to them by clasps 15,19.

Another advantage of the device 1 of the present invention is that it can be adjusted to improve fit and retention and it can also be reactivated or deactivated without the need to remake the whole device.

A further advantage is that regular wear of the device 1 can allow it to act as an orthodontic tooth retainer. It is also helpful in discouraging patients from grinding their teeth during wear and sleep.

## Claims

1. A device (1) for advancing a lower jaw (5) relative to an upper jaw (4) in a mouth, the device comprising:
upper and lower members (2, 3) arranged to be positioned on upper and lower jaws respectively and connected by connection means (21):
the upper member (2) being arranged to be positioned behind the upper front teeth (17) in the upper jaw and configured to extend back towards the upper molars (18) on either side of the upper jaw, the upper member having retaining means (19) for retaining the upper member within the upper jaw by engagement with one or more of the upper molars;
the lower member (3), being arranged to be positioned forward of the lower front teeth (9) in the lower jaw (5) and configured to extend backwards to cover at least part of the occlusal surfaces of one or more of the lower molars (11) on either side of the lower jaw, the lower member having retaining means (15) for retaining it within the lower jaw by engagement with one or more of the lower molars, and
the connection means (21) being configured to removably connect the upper member (2) to the lower member (3) such that the lower jaw (5) is advanced to and retained at a predetermined minimum distance relative to the upper jaw (4), **characterised in that** the lower member is arranged so as not to extend behind the lower front teeth.

2. The device according to claim 1, wherein the connection means (21) comprises a telescopic arm (6) and sleeve (7).

3. The device according to claim 2, wherein one of the arm (6) or sleeve (7) is pivotally attached at one of its ends to a rearward portion of the upper member (2) and the other of the arm (6) or sleeve (7) is pivotally attached at one of its ends to a forward portion of the lower member (3), the unattached end of the arm (6) being configured to removably fit inside the unattached end of the sleeve (7).

4. The device according to claim 2 or 3, wherein the arm (6) and sleeve (7) are pivotally attached to the upper and lower members (2,3) by grub screws (13).

5. The device according to any preceding claim, wherein the upper member (2) is arranged to cover at least part of one or more of the upper molars (18) on either side of the upper jaw (4).

6. The device according to any preceding claim, wherein the upper member (2) is moulded to a predefined shaped to fit behind the upper teeth (17,18).

7. The device according to any preceding claim, wherein the retaining means (15,19) are configured to secure the upper and lower members (2,3) to the lingual and buccal sides of one or more of the upper and lower molars (18, 11).

8. The device according to any preceding claim, wherein the upper and lower members (2,3) are provided with mounting points (20,12) for attaching the connecting means (21).

9. The device according to any preceding claim, wherein the upper member (2) or lower member (3) is formed from a cast metal.

10. The device according to claim 9, wherein the cast metal is a gold alloy.

11. The device according to claim 9, wherein the cast metal is chrome cobalt.

## Patentansprüche

1. Vorrichtung (1) zum Verschieben eines Unterkiefers (5) im Verhältnis zu einem Oberkiefer (4) in einem Mund, wobei die Vorrichtung umfasst:
oberes und unteres Element (2,3), die zum Positionierten auf dem Ober- bzw. Unterkiefer angeordnet sind und durch Verbindungsmittel (21) verbunden sind,
wobei das obere Element (2) zum Positionierten hinter den oberen Vorderzähnen (17) im Oberkiefer angeordnet ist und so konfiguriert ist, dass as sich zurück in Richtung auf die oberen Backenzähne (18) auf beiden Seiten des Oberkiefers erstreckt, wobei das obere Element Haltemittel (19) aufweist, um das obere Element durch Eingriff mit einem oder mehr der oberen Backenzähne im Oberkiefer zu halten;
wobei das untere Element (3) zum Positionierten vor den unteren Vorderzähnen (9) im Unterkiefer (5) angeordnet ist und so konfiguriert ist, dass es sich rückwärts erstreckt, um mindestens einen Teil der Okklusionsflächen von einem oder mehr der unteren Backenzähne (11) auf beiden Seiten des Unterkiefers abzudecken, wobei das untere Element Haltemittel (15) aufweiset, um es durch Eingriff mit einem oder mehr der unteren Backenzähne im Unterkiefer zu halten; und
wobei das Verbindungsmittel (21) so konfiguriert ist, dass es das obere Element (2) entfernbar mit dem unteren Element (3) verbindet, so dass der Unterkiefer (5) zum Oberkiefer (4) vorgeschoben und in einem vorbestimmten Mindestabstand zu diesem gehalten wird, **gekennzeichnet dadurch dass** das untere Element so angeordnet ist, dass es sich nicht hinter den unteren Vorderzähnen erstreckt.

2. Vorrichtung nach Anspruch 1, wobei das Verbindungsmittel (21) einen Teleskoparm (6) und eine Hülse (7) umfasst.

3. Vorrichtung nach Anspruch 2, wobei das eine des Arms (6) oder der Hülse (7) schwenkbar an einem seiner Enden an einem rückwärtigen Teil des oberen Elements (2) befestigt ist und das andere des Arms (6) oder der Hülse (7) schwenkbar an einem seiner Enden an einem vorderen Teil des unteren Elements (3) befestigt ist, wobei das nicht befestigte Ende des Arms (6) so konfiguriert ist, dass es entfernbar in das Innere des nicht befestigten Endes der Hülse (7) hineinpasst.

4. Vorrichtung nach Anspruch 2 oder 3, wobei der Arm (6) und die Hülse (7) schwenkbar Madenschrauben (13) an dem oberen und dem unteren Element (2, 3) befestigt sind.

5. Vorrichtung nach einem der vorausgehenden Ansprüche, wobei das obere Element (2) so angeordnet ist, **dass** es mindestens einen Teil von einem oder mehr der oberen Backenzähne (18) auf jeder Seite des Oberkiefers (4) abdeckt.

6. Vorrichtung nach einem der vorausgehenden Ansprüche, wobei das obere Element (2) zu einer vorbestimmten Gestalt geformt ist, um hinter die oberen Zähne zu passen (17, 18).

7. Vorrichtung nach einem der vorausgehenden Ansprüche, wobei die Haltemittel (15, 19) so konfiguriert sind, **dass** sie das obere und untere Element (2, 3) an den der Zunge und Mundhöhle zugewandten Seiten von einer oder mehr der oberen und unteren Backenzähne (18, 11) sichern.

8. Vorrichtung nach einem der vorausgehenden Ansprüche, wobei das obere und untere Element (2, 3) mit Befestigungspunkten (20, 12) zum Befestigen der Verbindungsmittel (21) ausgestattet sind.

9. Vorrichtung nach einem der vorausgehenden Ansprüche, wobei das obere Element (2) oder das untere Element (3) aus einem Gussmetall geformt ist.

10. Vorrichtung nach Anspruch 9, wobei das Gussmetall eine Goldlegierung ist.

11. Vorrichtung nach Anspruch 9, wobei das Gussmetall Chrom-Kobalt ist.

## Revendications

1. Dispositif (1) pour avancement d'une mâchoire inférieure (5) relativement à une mâchoire supérieure (4) dans une bouche, le dispositif comprenant :
des éléments supérieur et inférieur (2, 3) conçus pour être positionnés sur les mâchoires supérieure et inférieure respectivement et raccordés par un moyen de connexion (21)
l'élément supérieur (2) étant conçu pour être positionné derrière les dents supérieures avant (17) dans la mâchoire supérieure et configuré pour s'étendre vers l'arrière en direction des molaires supérieures (18) de chaque côté de la mâchoire supérieure, l'élément supérieur présentant un moyen de retenue (19) pour maintenir l'élément supérieur dans la mâchoire supérieure par engagement avec une ou plusieurs des molaires supérieures ;
l'élément inférieur (3) étant conçu pour être positionné en avant des dents inférieures avant (9) dans la mâchoire inférieure (5) et configuré pour s'étendre vers l'arrière afin de couvrir au moins une partie des surfaces occlusales d'une ou de plusieurs des molaires inférieures (11) de chaque côté de la mâchoire inférieure, le membre inférieur ayant un moyen de retenue (15) pour le maintenir dans la mâchoire inférieure par engagement avec une ou plusieurs des molaires inférieures,, et
le moyen de connexion (21) étant configuré pour relier de façon amovible l'élément supérieur (2) à l'élément inférieur (3) de manière à ce que la mâchoire inférieure (5) soit avancée vers et maintenue à une distance minimale prédéterminée relativement à la mâchoire supérieure (4), **caractérisé en ce que** l'élément inférieur est placé de manière à s'étendre derrière les dents inférieures avant.

2. Dispositif selon la revendication 1, dans lequel le moyen de connexion (21) comprend un bras télescopique (6) et un manchon (7).

3. Dispositif selon la revendication 2, dans lequel un bras (6) ou un manchon (7) est fixé pivotant à l'une de ses extrémités à une partie arrière de l'élément supérieur (2) et l'autre du bras (6) ou du manchon (7) est fixé pivotant à l'une de ses extrémités à une partie avant de l'élément inférieur (3), l'extrémité non fixée du bras (6) étant configurée pour tenir de façon amovible à l'intérieur de l'extrémité non fixée du manchon (7).

4. Dispositif selon la revendication 2 ou 3, dans lequel le bras (6) et le manchon (7) sont fixés pivotants aux éléments supérieur et inférieur (2,3) par des vis d'arrêt (13).

5. Dispositif selon l'une quelconque des revendications précédentes, dans lequel l'élément supérieur (2) est placé de façon à couvrir au moins une partie ou plus des molaires supérieures (18) de chaque coté de la mâchoire supérieure (4).

6. Dispositif selon l'une quelconque des revendications précédentes, dans lequel l'élément supérieur (2) est moulé en une forme prédéfinie pour tenir derrière les dents supérieures (17, 18).

7. Dispositif selon une quelconque revendication précédente, dans lequel les moyens de retenue (15, 19) sont configurés pour fixer les éléments supérieur et inférieur (2,3) aux côtés lingual et buccal d'une ou de plusieurs des molaires supérieures et inférieures (18, 11).

8. Dispositif selon l'une quelconque revendication précédente, dans lequel les éléments supérieur et inférieur (2, 3) sont prévus avec des points de montage (20, 12) aux fins de fixation du moyen de connexion (21).

9. Dispositif selon une quelconque revendication précédente, dans lequel l'élément supérieur (2) ou l'élément inférieur (3) est formé de métal coulé.

10. Dispositif selon la revendication 9, dans lequel le métal coulé est un alliage d'or.

11. Dispositif selon la revendication 9, dans lequel le métal coulé est du chrome de cobalt.
